(19) Europäisches Patentamt
European Patent Office
Office européen des brevets

(11) EP 2 376 510 B1

(12) # FASCICULE DE BREVET EUROPEEN

(45) Date de publication et mention de la délivrance du brevet:
**03.12.2014 Bulletin 2014/49**

(21) Numéro de dépôt: **09803842.5**

(22) Date de dépôt: **11.12.2009**

(51) Int Cl.:
***C07H 7/04*** *(2006.01)* ***A61K 8/60*** *(2006.01)*
***A61Q 19/00*** *(2006.01)* ***C07D 309/10*** *(2006.01)*
***C07D 407/10*** *(2006.01)* ***A61K 31/70*** *(2006.01)*
***A61P 17/00*** *(2006.01)*

(86) Numéro de dépôt international:
**PCT/FR2009/052499**

(87) Numéro de publication internationale:
**WO 2010/067036 (17.06.2010 Gazette 2010/24)**

(54) **NOUVEAUX COMPOSÉS C-XYLOSIDES ARYLE ET UTILISATION EN COSMÉTIQUE**

NEUE ARYL-C-XYLOSIDVERBINDUNGEN UND KOSMETISCHE VERWENDUNG

NOVEL ARYL C-XYLOSIDE COMPOUNDS, AND COSMETIC USE

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **12.12.2008 FR 0858537**
**23.12.2008 US 140294 P**

(43) Date de publication de la demande:
**19.10.2011 Bulletin 2011/42**

(73) Titulaire: **L'Oréal**
**75008 Paris (FR)**

(72) Inventeurs:
- **DALKO, Maria**
**F-78000 Versailles (FR)**
- **CAVEZZA, Alexandre**
**F-93320 Pavillon Sous Bois (FR)**

(74) Mandataire: **Kromer, Christophe**
**L'Oréal**
**D.I.P.I.**
**25-29 Quai Aulagnier**
**92600 Asnières (FR)**

(56) Documents cités:
**WO-A-02/051828**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 376 510 B1

Printed by Jouve, 75001 PARIS (FR)

## Description

La présente invention concerne de nouveaux dérivés C-xylosides aryles, les compositions notamment cosmétiques les comprenant, ainsi que leur utilisation pour lutter contre le vieillissement cutané.

Les femmes et les hommes ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en améliorant son renouvellement cellulaire ou encore en favorisant la synthèse, ou en prévenant la dégradation, des fibres élastiques qui composent le tissu cutané.

On sait que la peau humaine est constituée de deux tissus l'un superficiel, l'épiderme, l'autre profond, le derme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures (climat, rayons ultraviolets, tabac, ...), appelé "fonction barrière".

Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes. On y trouve aussi des leucocytes, des mastocytes ou encore des macrophages tissulaires. Enfin, le derme est traversé par des vaisseaux sanguins et des fibres nerveuses.

La matrice extracellulaire du derme est composée de protéines appartenant à plusieurs grandes familles : les collagènes, les glycoprotéines matricielles autres que les collagènes (fibronéctine, laminine), l'élastine et les protéoglycannes. On trouve également dans la matrice extracellulaire du derme des glycosaminoglycannes sous forme libre (c'est à dire non liés à une protéine).

Il est maintenant bien établi que des intéractions spécifiques existent entre ces différentes classes de protéines pour donner naissance à un tissu fonctionnel. Un espace extracellulaire (micromatrice) existe également dans l'epiderme. Cet espace joue un rôle fonctionnel extrêmement important dans le renouvellement et ou le maintien du tissu cellulaire.

Les protéoglycannes sont des macromolécules complexes constituées d'un tronc protéique central ramifié, ou réseau de protéines, auquel sont attachés de très nombreuses chaînes latérales polyosidiques appelées glycosaminoglycannes.

Dans la suite de la présente demande, on désignera les protéoglycannes par l'abréviation PGs et les glycosaminoglycannes par l'abréviation GAGs.

Les GAGs ont longtemps été désignés sous le terme de mucopolysaccharides acides en raison de leur forte capacité de rétention d'eau, de leur nature glucidique et de leur caractère acide provenant de leurs multiples charges négatives.

Ainsi, la polarité des GAGs les fait implicitement participer à certaines fonctions biologiques comme l'hydratation des tissus, la fixation des cations ou le rôle de barrière de filtration ionique.

Les PGs et les GAGs sont synthétisés par différentes cellules au niveau du derme et de l'épiderme: fibroblastes, kératinocytes et mélanocytes.

Les fibroblastes synthétisent majoritairement des collagènes, des glycoprotéines matricielles autres que les collagènes (fibronéctine, laminine), des GAGs, des protéoglycannes et de l'élastine. Les kératinocytes synthétisent majoritairement des GAGs sulfatés et de l'acide hyaluronique alors que les mélanocytes ne produisent apparemment pas d'acide hyaluronique.

Lors de leur incorporation dans un PG, les GAGs sont sous la forme de chaînes linéaires composées de la répétition d'un diholoside de base contenant toujours une hexosamine (glucosamine ou galactosamine) et un autre ose (acide glucuronique, acide iduronique ou galactose). La glucosamine est soit N-sulfatée, soit N-acétylée. En revanche, la galactosamine est toujours N-acétylée. En outre, il peut y avoir des groupes sulfates O-liés sur l'hexosamine, l'acide uronique et le galactose.

Le fort caractère anionique des GAGs s'explique par la présence de groupes carboxylates au sein des acides hexuroniques (acide glucuronique et acide iduronique) et de groupes sulfate O- et N-liés.

Les principaux GAGs sont l'acide hyaluronique ou hyaluronanne (HA), l'héparane sulfate (HS), l'héparine (HP), la chondroïtine, la chondroïtine sulfate

(CS), la chondroïtine 4-sulfate ou chondroïtine sulfate A (CSA), la chondroïtine 6-sulfate ou chondroïtine sulfate C (CSC), le dermatane sulfate ou chondroïtine sulfate B (CSB) et le kératane sulfate (KS) qui diffère des autres glycosaminoglycannes par la présence de galactose à la place de l'acide uronique.

Les protéoglycanes (PGs) sont formés par l'ancrage de plusieurs chaînes de GAGs sur une chaîne polypeptidique (dite protéine porteuse ou protéine « core »).

Les GAGs peuvent également exister dans la matrice extracellulaire sous forme libre c'est à dire non liée à une protéine matricielle : c'est notamment le cas de l'acide hyaluronique.

Lors de la synthèse des PGs, les GAGs sont polymérisés à partir de ces structures d'ancrage.

La synthèse des GAGs nécessite l'action coordonnée et concertée d'enzymes très spécifiques (transférases, épimérases, sulfotransférases) adjacentes dans la membrane du réticulum endoplasmique et de l'appareil de golgi. Puis une multitude de réactions biochimiques (N-désacétylation, N- et O-sulfatations, épimérisation) modifient les deux oses constitutifs du motif de base et cela de manière hétérogène le long de la chaîne. D'une chaîne d'héparane sulfate à l'autre par exemple, le rapport acide glucuronique/acide iduronique, la nature, le nombre et la position des O-sulfatations, ainsi que le rapport N-sulfate/O-sulfate peuvent varier, ce qui, potentiellement offre une immense diversité structurale.

De façon générale, les rôles biologiques des PGs sont très diversifiés, allant d'une fonction passive de support mécanique (par exemple serglycines) ou d'un rôle de barrière ionique de filtration moléculaire (par exemple perlecanne et bamacanne de la membrane basale glomérulaire), à des effets plus spécifiques dans l'adhésion, l'étalement, la prolifération, la différenciation cellulaire ou la morphogenèse, ou, à des effets très spécifiques d'interactions PG-protéine, tels que la fonction de récepteur du bétaglycanne ou l'interaction de la décorine avec le collagène. Ils jouent également un rôle fondamental dans la libération contrôlée de différents facteurs de croissance.

Un des rôles du tissu conjonctif dermique est de protéger l'organisme contre les agressions externes en formant en même temps une interface informative.

Pour ce faire, le derme possède une forte résistance mécanique en gardant, toutefois, une grande souplesse.

Sa résistance est assurée par le réseau dense des fibres de collagène, mais ce sont les PGs et l'acide hyaluronique, en assurant l'hydratation, la distribution et la souplesse des fibres qui font la différence entre la peau et, par exemple, le cuir.

Les PGs constituent 0,5 à 2 % du poids sec du derme, le collagène en représentant à lui seul jusqu'à 80 %.

La concentration et la distribution dans la peau humaine des GAGs et des PGs varient avec l'âge.

L'acide hyaluronique ou hyaluronanne (HA) est le principal GAG du derme, ce dernier renfermant la moitié d'HA de l'organisme.

La synthèse d'HA est effectuée notamment par les fibroblastes, à proximité de la face interne de la membrane plasmique. Elle s'effectue de façon continue.

Ce polysaccharide gigantesque (plusieurs millions de daltons) possède une viscosité intrinsèque très élevée, assurant l'hydratation et l'assemblage des différents éléments du tissu conjonctif par formation de complexes supramoléculaires.

Le dermatanne sulfate (DS), initialement isolé du derme, est aussi très abondant dans la peau. Il constitue 40 à 50 % des GAGs dermiques.

Parallèlement aux mécanismes contribuant à l'élaboration de ces matrices extracellulaires spécialisées, il existe des processus de remodelage continuel dont la régulation dépend de la balance entre synthèse et dégradation des éléments protéiques de la matrice.

Plusieurs familles de protéases matricielles sont maintenant décrites ainsi que les facteurs intervenant dans leur activation-inactivation.

Au cours du vieillissement chronologique et/ou photoinduit, le derme et l'épiderme subissent de nombreuses modifications et dégradations qui se traduisent, avec l'âge, par une flaccidité et une perte de souplesse cutanée.

Parmi les éléments dégradés (notamment collagène et élastine), les PGs et les GAGs sont également altérés. En effet, au cours du vieillissement, les fibroblastes et les kératinocytes produisent de moins en moins de PGs et de GAGs et leur synthèse est imparfaite. Il en résulte une désorganisation importante : le dépôt des GAGs sur le squelette protéique formant le PG est anormal, ce qui a pour conséquence, une moins grande avidité pour l'eau de ces PGs et donc une diminution de l'hydratation et de la tonicité des tissus.

Restaurer une production normale de PGs et de GAGs par les fibroblastes et les kératinocytes contribue, en partie, à compenser la perte en hydratation cutanée.

La dégradation de ces matrices contribue donc au phénomène d'assèchement et de perte de souplesse de la peau.

On comprend donc l'importance de pouvoir disposer de produits dont les effets visent à maintenir le taux de PGs et de GAGs dans la peau et lui maintenir ainsi entre autre une bonne hydratation et une bonne souplesse.

Il est connu du document WO 02/051828 d'utiliser des composés C-glycosides pour augmenter la synthèse des glycosaminoglycannes par les fibroblastes et/ou les kératinocytes.

Il est également connu du document WO 2006/128738 d'autres composés C-glycosides pour dépigmenter la peau.

La demanderesse a découvert, de manière surprenante et inattendue, que d'autres dérivés C-glycosides aryles sont capables d'améliorer la synthèse des glycosaminoglycannes sulfatés comme la chondroïtine sulfate et le dermatane sulfate.

Leur efficacité est plus performante que celle des composés C-glycosides déjà connus. Ils sont plus efficaces pour améliorer le renouvellement épidermique et la fermeté de la peau et pour lutter plus efficacement contre les signes du vieillissement cutané. Ils ont également un effet bénéfique sur la structure de la jonction dermo-épidermique, notamment sur la cohésion entre derme et épiderme.

Ces nouveaux composés trouvent donc une application particulière dans les compositions cosmétiques ou pharmaceutiques, en particulier dermatologiques, destinées à prévenir et/ou traiter cosmétiquement le vieillissement cutané; notamment prévenir et/ou traiter, en particulier par voie topique, les signes cutanés du vieillissement, et tout particulièrement les signes cutanés liés à une peau ridée, une peau présentant une altération de ses propriétés viscoélastiques ou biomécaniques, une peau présentant une altération dans la cohésion de ses tissus, une peau amincie et/ou une peau présentant une altération de son aspect de surface.

Les compositions selon l'invention peuvent permettre plus particulièrement de maintenir et/ou de restaurer les propriétés d'extensibilité, de tonicité, de fermeté, de souplesse, de densité et/ou d'élasticité de la peau.

Par "propriétés biomécaniques de la peau", on entend ici les propriétés d'extensibilité, de tonicité, de fermeté, de souplesse et/ou d'élasticité de la peau.

Par "signes cutanés du vieillissement", on entend ici toute modification de l'aspect extérieur de la peau due au vieillissement qu'il soit chronobiologique et/ou extrinsèque, en particulier photoinduit ou hormonal; parmi ces signes, on peut distinguer:

- la peau ridée, qui se traduit notamment par l'apparition de rides et/ou ridules,
- la peau présentant une altération de ses propriétés viscoélastiques ou biomécaniques, ou peau présentant un manque d'élasticité et/ou d'extensibilité et/ou de fermeté et/ou de souplesse et/ou de tonicité, qui se traduit notamment par une peau flétrie, molle, relâchée ou affaissée,
- la peau présentant une altération de la cohésion de ses tissus;
- la peau amincie,
- la peau présentant une altération de son aspect de surface, qui se traduit notamment par une altération du grain de la peau, par exemple une rugosité.

La présente invention a donc pour objet de nouveaux composés C-glycosides aminés de formule (I) telle que définie ci-après.

L'invention concerne également une composition cosmétique ou pharmaceutique comprenant, dans un milieu physiologiquement acceptable, au moins un tel composé.

Les composés selon l'invention répondent donc à la formule (I) suivante :

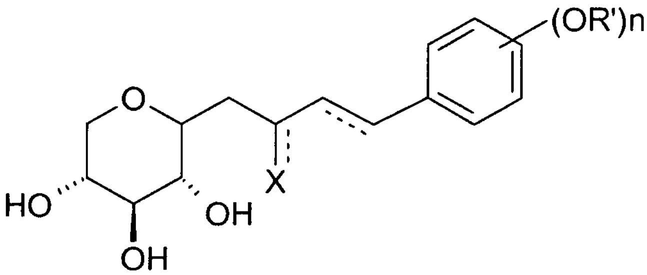

dans laquelle :

X désigne un groupement -OH ou =O ;
n est un entier allant de 0 à 5 ;
R' désigne :

un atome d'hydrogène ou
un radical alkyle linéaire en $C_1$-$C_4$ ou ramifié en $C_3$-$C_4$, ou un radical hydrocarboné insaturé en $C_3$-$C_4$ ,
lorsque n ≥ 2 , deux groupements adjacents -OR' peuvent former ensemble radical divalent $-O-CH_2-O-$ ;
sous réserve que lorsque X = OH, le composé ne comporte pas de double liaison éthylénique en position alpha du carbone portant cet OH ;
ainsi que leurs sels, et leurs isomères optiques.

Le terme alkyle, dans le cadre de la présente invention, signifie une chaîne hydrocarbonée saturée ou insaturée. Parmi les groupes alkyle convenant à la mise en oeuvre de l'invention, on peut notamment citer les groupes méthyle,

éthyle, isopropyle, n-propyle, n-butyle, t-butyle, isobutyle, sec-butyle, pentyle, hexyle, heptyle, octyle, nonyle, décyle.

De préférence,

X désigne un groupement -OH ou =O ;
n est un entier allant de 0 à 3 ;
R' désigne :

un atome d'hydrogène ou
un radical alkyle linéaire en $C_1$-$C_4$ , notamment un radical méthyle ou n-butyle ;
ou un radical $-CO-CH_3$ ;
lorsque n ≥ 2 , deux groupements adjacents -OR' peuvent former ensemble radical divalent $-O-CH_2-O-$ ;

Préférentiellement :

X désigne un groupement -OH ou =O ;
n est un entier allant de 0 à 3 ;
R' désigne :

un atome d'hydrogène ou
un radical méthyle.

En particulier, n et R' peuvent être tels que la partie aromatique

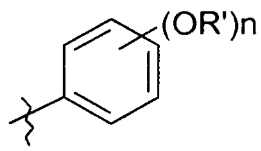

des composés de formule (I°) peut correspondre à l'une des structures suivantes :

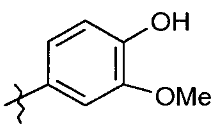 , 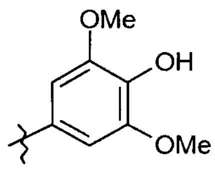 , 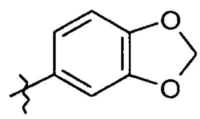 , 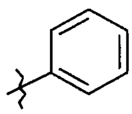

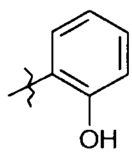
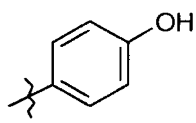

On peut citer en particulier les composés de formule (I) suivants (y compris leurs sels ou leurs solvates) :

- 1-(α,β)-D-xylopyranosyl, 4-(4-hydroxy,3-méthoxy-phenyl) butane-2-ol (composé 1) :

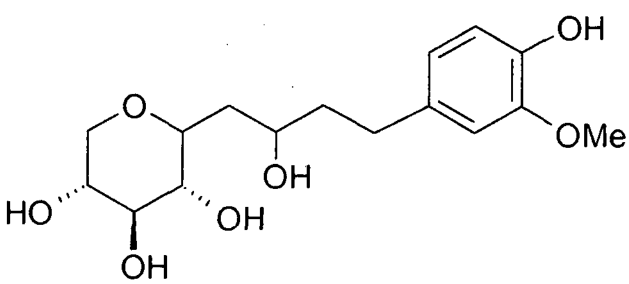

- 1-(α,β)-D-xylopyranosyl, 4-(4-hydroxy,3-méthoxy-phényl) butane-2-one (composé 2) :

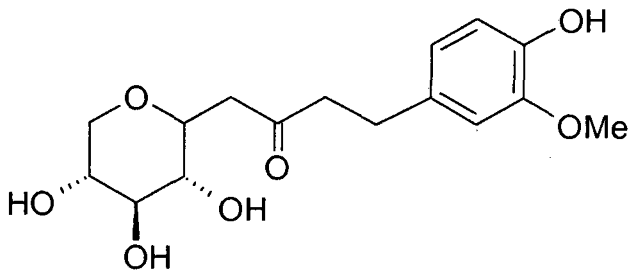

- 1-(α,β)-D-xylopyranosyl, 4-(4-hydroxyphényl) butane-2-ol (composé 3) :

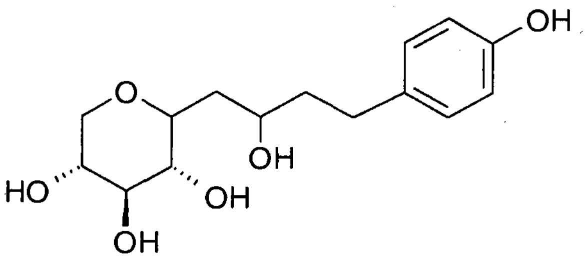

- 1-(α,β)-D-xylopyranosyl, 4-phényl butane-2-one (composé 4)

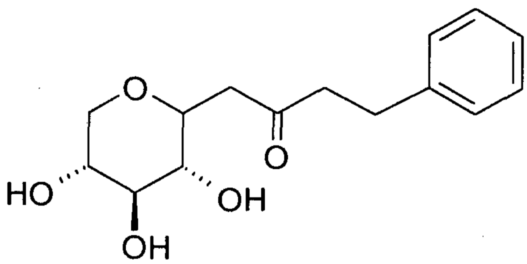

- (3E)-1-(α,β)-D-xylopyranosyl, 4-phényl but-3-ène-2-one (composé 5) :

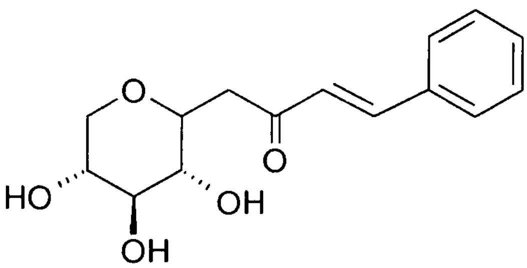

- (3E)-1-(α,β)-D-xylopyranosyl, 4-[4-hydroxy, (3,5)-diméthoxy-phényl] but-3-ène-2-one (composé 6) :

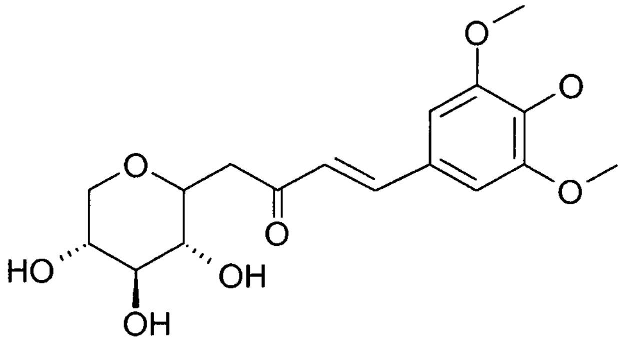

- 1-(α,β)-D-xylopyranosyl, 4-[(3,4)-méthylènedioxyphenyl] butane-2-ol (composé 7) :

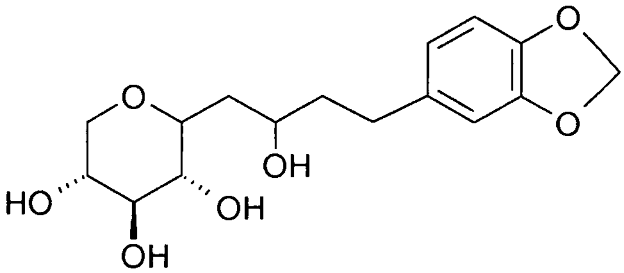

- 1-(α,β)-D-xylopyranosyl, 4-(2-hydroxyphényl) butane-2-ol (composé 8) :

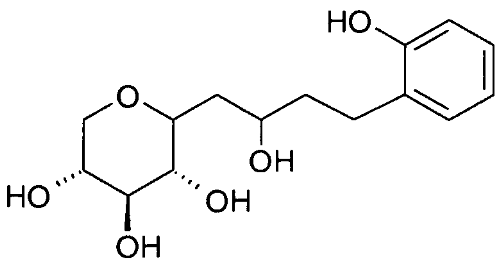

- 1-(α,β)-D-xylopyranosyl, 4-phényl butane-2-ol (composé 9) :

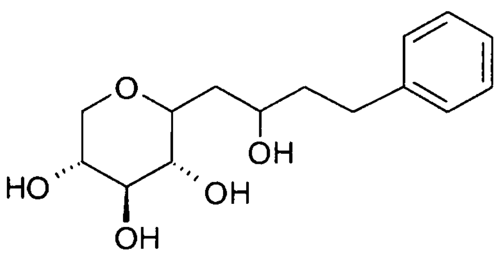

Les solvates acceptables des composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

Les composés de formule (I) peuvent être préparés selon le schéma I ci-après :

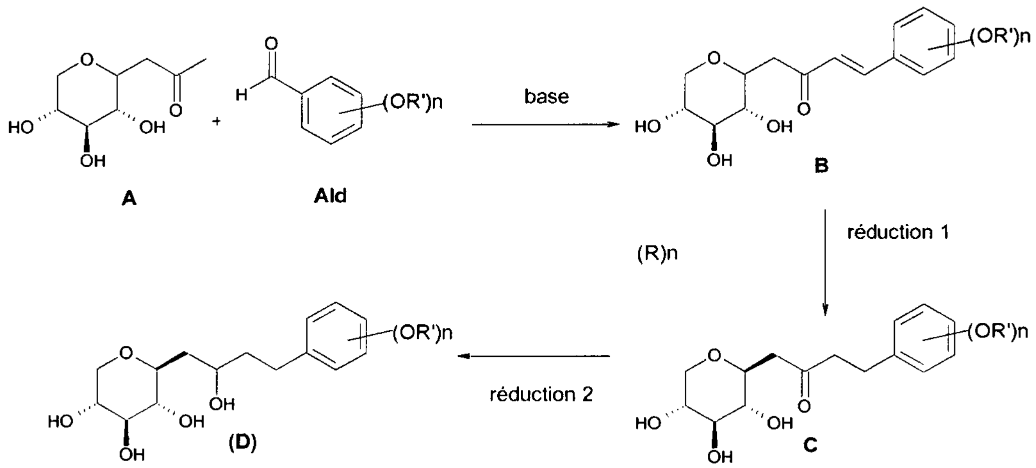


A Ald B C (D)

par réaction de la C- -D-xylopyranoside-n-propane-2-one (A) (composé décrit à l'exemple 1 de la demande WO 02/051828) avec 1 à 2 équivalents de benzaldéhyde de formule (III) (Ald) dans laquelle n et R' ont les significations telles que décrites précédemment dans les composés de formule (I), notamment à la température ambiante (25 °C), dans un solvant polaire tel que l'eau, l'éthanol; l'isopropanol, le méthanol, en présence d'une base (par exemple la soude ou le méthylate de sodium), notamment pendant 1 à 20 heures.

Le produit d'aldolisation (B) obtenu peut ensuite être réduit partiellement de façon connue par hydrogénation catalytique (par exemple Pd/C / $H_2$) pour obtenir le composé (C). Le composé (C) peut à son tour être réduit de façon connue à l'aide d'un hydrure (par exemple borohydrure de sodium) ou bien par hydrogénation catalytique avec du palladium sur charbon suivi d'une étape d'hydrogénation en présence d'hydrogène sous pression, pour obtenir le composé (D).

Les composés B, C, D sont des composés conforme à la structure de formule (I) décrite précédemment.

La présente invention concerne également une composition comprenant, dans un milieu physiologiquement acceptable, un composé de formule (I) tel que décrit ci-dessus. La composition est en particulier une composition cosmétique ou dermatologique.

Le composé de formule (I) peut être présent dans les compositions cosmétiques ou pharmaceutiques, en une quantité qui peut être comprise entre 0,01 et 10% en poids, de préférence entre 0,1 à 5% en poids, notamment entre 0,5 à 3% en poids, par rapport au poids total de la composition.

La composition comprend en outre un milieu physiologiquement acceptable, qui sera préférentiellement un milieu cosmétiquement ou pharmaceutiquement, notamment dermatologiquement, acceptable, c'est-à-dire sans odeur, couleur ou aspect désagréable, et qui ne génère pas de picotement, tiraillement ou rougeur inacceptable pour l'utilisateur. En particulier la composition est adaptée à une application topique sur la peau.

Par milieu physiologiquement acceptable, on comprend un milieu compatible avec les matières kératiniques d'êtres humains comme la peau du corps ou du visage, les lèvres, les muqueuses, les cils, les ongles, le cuir chevelu et/ou les cheveux.

La composition selon l'invention peut alors comprendre tous les adjuvants cosmétiques usuellement employés dans le domaine d'application envisagée.

On peut notamment citer l'eau; les solvants organiques, notamment les alcools en $C_1$-$C_6$ et les esters d'acide carboxylique en $C_2$-$C_{10}$; les huiles hydrocarbonées, les huiles siliconées, les huiles fluorées, les cires, les pigments, les charges, les colorants, les tensioactifs, les émulsionnants, les actifs cosmétiques ou dermatologiques, les filtres UV, les polymères filmogènes, les gélifiants hydrophiles ou lipophiles, les épaississants, les conservateurs, les parfums, les bactéricides, les absorbeurs d'odeur, les antioxydants.

Ces éventuels adjuvants peuvent être présents dans la composition à raison de 0,001 à 80% en poids, notamment 0,1 à 40% en poids, par rapport au poids total de la composition.

Ces adjuvants, selon leur nature, peuvent être introduits dans la phase grasse ou dans la phase aqueuse de la composition, ou dans des vésicules lipidiques. En tout état de cause, ces adjuvants, ainsi que leurs proportions, seront choisis par l'homme du métier de manière telle que les propriétés avantageuses des composés selon l'invention ne soient pas, ou substantiellement pas, altérées par l'adjonction envisagée.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme gélifiants ou épaississants hydrophiles, on peut citer les polymères carboxyvinyliques (carbomer), les

copolymères acryliques tels que les copolymères d'acrylates/alkylacrylates, les polyacrylamides, les polysaccharides, les gommes naturelles et les argiles; comme gélifiants ou épaississants lipophiles, on peut citer les argiles modifiées comme les bentones, les sels métalliques d'acides gras, et la silice hydrophobe.

Comme actifs, il sera avantageux d'introduire dans la composition utilisée selon l'invention au moins un composé choisi parmi: les agents desquamants; les agents hydratants; les agents dépigmentants ou propigmentants; les agents anti-glycation; les inhibiteurs de NO-synthase; les agents stimulant la synthèse de macromolécules dermiques ou épidermiques et/ou empêchant leur dégradation; les agents stimulant la prolifération des fibroblastes et/ou des kératinocytes ou stimulant la différenciation des kératinocytes; les agents myorelaxants et/ou les agents dermo-décontractants; les agents tenseurs; les agents anti-pollution et/ou anti-radicalaire; les agents agissant sur la microcirculation; les agents agissant sur le métabolisme énergétique des cellules; et leurs mélanges.

Des exemples de tels composés additionnels sont: le rétinol et ses dérivés tels que le palmitate de rétinyle; l'acide ascorbique et ses dérivés tels que l'ascorbyl phosphate de magnésium et le glucoside d'ascorbyle; le tocophérol et ses dérivés tels que l'acétate de tocophéryle; l'acide nicotinique et ses précurseurs tels que la nicotinamide; l'ubiquinone; le glutathion et ses précurseurs tels que l'acide L-2-oxothiazolidine-4-carboxylique; les extraits de plantes et notamment les protéines végétales et leurs hydrolysats, ainsi que les phytohormones; les extraits marins tels que les extraits d'algues; les extraits bactériens; les sapogénines telles que la diosgénine et les extraits de Wild Yam en contenant; les céramides; les hydroxyacides tels que l'acide salicylique et l'acide n-octanoyl-5-salicylique; le resvératrol; les oligopeptides et pseudodipeptides et leurs dérivés acylés; les sels de manganèse et de magnésium, en particulier les gluconates; et leurs mélanges.

Comme indiqué précédemment, la composition selon l'invention peut également renfermer des filtres UV ou agents photoprotecteurs actifs dans l'UVA et/ou l'UVB, sous forme de composés organiques ou inorganiques, ces derniers étant éventuellement enrobés pour les rendre hydrophobes.

Les agents photoprotecteurs organiques peuvent notamment être choisis parmi: les anthranilates, en particulier l'anthranilate de menthyle; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-3 (Oxybenzone), ou la Benzophénone-4 (Uvinul MS40 de BASF); les benzylidènes-camphres, en particulier le 3-benzylidène-camphre, l'acide benzylidènecampho-sulfonique, le benzalkoniumméthosulfate de camphre, le polyacrylamidométhylbenzylidène camphre, l'acide téréphthalylidène di-camphre sulfonique, et préférentiellement le 4-méthylbenzylidène camphre (Eusolex 6300 de Merck); les benzimidazoles, en particulier le benzimidazilate (Neo Heliopan AP de Haarmann et Reimer), ou l'acide phénylbenzimidazole sulfonique (Eusolex 232 de Merck); les benzotriazoles, en particulier le drométrizole trisiloxane, ou le méthylène bis-benzotriazolyltétraméthylbutylphénol (Tinosorb M de Ciba); les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35 de BASF), l'octylméthoxycinnamate (Parsol MCX de Hoffmann La Roche), ou l'octocrylène (Uvinul 539 de BASF); les dibenzoylméthanes, en particulier le butyl méthoxydibenzoylméthane (Parsol 1789); les imidazolines, en particulier l'éthylhexyl diméthoxybenzylidène dioxoimidazoline; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB de 3V Sigma), l'éthylhexyltriazone (Uvinul T150 de BASF), ou l'éthyl PABA (benzocaïne); les salicylates, en particulier le salicylate de dipropylèneglycol, le salicylate d'éthylhexyle, l'homosalate, ou le TEA salicylate; les triazines, en particulier l'anisotriazine (Tinosorb S de Ciba); le drometrizole trisiloxane.

Les agents photoprotecteurs inorganiques sont de préférence constitués d'oxyde de zinc et/ou de dioxyde de titane, de préférence de taille nanométrique, éventuellement enrobés d'alumine et/ou d'acide stéarique.

Cette composition peut se présenter sous toutes les formes galéniques normalement utilisées dans le domaine cosmétique ou pharmaceutique, et notamment sous forme d'une solution aqueuse ou hydroalcoolique, éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique; de gel aqueux ou huileux. Ces compositions sont préparées selon les méthodes usuelles. On préfère utiliser selon cette invention une composition sous la forme d'une émulsion notamment huile-dans-eau.

La composition peut être plus ou moins fluide et avoir l'aspect d'une crème blanche ou colorée, d'une pommade, d'un lait, d'une lotion, d'un sérum, d'une pâte, d'un gel, d'une mousse. Elle peut éventuellement être appliquée sous forme d'aérosol. Elle peut également se présenter sous forme solide, en particulier sous forme de stick.

Lorsque la composition est une émulsion, la proportion de la phase grasse peut aller de 5 à 80% en poids, de préférence de 8 à 50% en poids par rapport au poids total de la composition. L'émulsionnant et le co-émulsionnant peuvent être présents en une proportion allant de 0,3 à 30% en poids, et de préférence de 0,5 à 20% en poids, par rapport au poids total de la composition.

La composition selon l'invention peut constituer une composition de soin de la peau, et notamment une crème de nettoyage, de protection, de traitement ou de soin pour le visage, pour les mains, pour les pieds, pour les grands

plis anatomiques ou pour le corps (par exemple crèmes de jour, crèmes de nuit, crèmes démaquillantes, crèmes de fond de teint, crèmes anti-solaires); un lait de démaquillage, un lait corporel de protection ou de soin, un lait anti-solaire; une lotion, gel ou mousse pour le soin de la peau, comme une lotion de nettoyage.

La composition selon l'invention est avantageusement une composition anti-âge, notamment de soin, destinée à traiter et/ou lutter contre, cosmétiquement, les signes extérieurs du vieillissement cutané; la composition est plus particulièrement une composition de soin des peaux matures.

La composition peut être également une composition de maquillage, notamment un fond de teint.

L'invention concerne également un procédé de traitement cosmétique de la peau, comprenant l'application sur la peau, d'une composition cosmétique telle que définie ci-dessus. Ce procédé trouve une application avantageuse dans le traitement de la peau, notamment de la peau mature et/ou de la peau ridée, en particulier du visage, notamment du front, du cou et/ou des mains.

L'invention concerne également l'utilisation cosmétique non thérapeutique d'une composition cosmétique telle que définie ci-dessus ou d'un composé de formule (I) tel que décrit précédemment, pour prévenir et/ou traiter les signes cutanés du vieillissement, notamment les signes cutanés choisis parmi la peau ridée, la peau présentant une altération de ses propriétés viscoélastiques ou biomécaniques, la peau présentant une altération dans la cohésion de ses tissus, la peau amincie, et la peau présentant une altération de son aspect de surface.

L'invention concerne aussi l'utilisation cosmétique non thérapeutique d'une composition telle que définie ci-dessus ou d'un composé de formule (I) tel que décrit précédemment, pour améliorer la fermeté de la peau et/ou pour améliorer la structure de la jonction dermo-épidermique et/ou pour renforcer la cohésion entre le derme et l'épiderme.

L'invention est illustrée plus en détail par les exemples non limitatifs suivants.

**Exemple 1 : Synthèse du - 1-(α,β)-D-xylopyranosyl, 4-(4-hydroxy,3-méthoxy-phényl) butane-2-one**

**(Composé 2)**

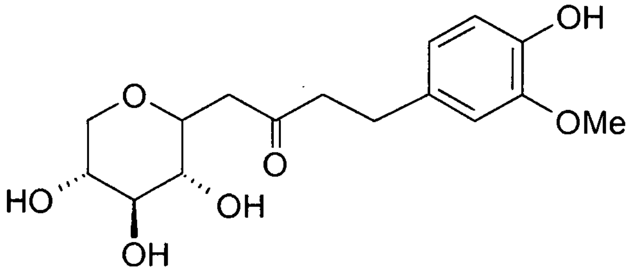

Dans 50 ml de méthanol anhydre, on a fait réagir 5 g (1 eq) de C-β-D-xylopyranoside-n-propane-2-one, avec du méthylate de sodium à 30% dans le méthanol (7.55 ml, 1.5 éq), puis on a ajouté 6.69 g (1.05 éq) de 4-benzyloxy-3-méthoxybenzaldehyde

Le milieu réactionnel a été agité pendant 15 heures à température ambiante (25 °C), puis a été dilué dans le dichlorométhane puis lavé avec une solution aqueuse de $NH_4Cl$. La phase organique aensuite été séchée sur sulfate de sodium puis concentrée sous vide. L'huile marron claire obtenue a été purifiée sur gel de silice pour obtenir l'intermédiaire (B) correspondant (solide jaune pâle, rendement de 30%).

L'intermédiaire réactionnel (B) obtenu (2 g, 1 éq) a été solubilisé dans un mélange eau/éthanol (5 ml/50 ml). Le palladium sur charbon à 10% (400 mg, 0.78 équivalent) a ensuite été ajouté ainsi que le cyclohexène (10 ml, 20.5 équivalents). Le milieu réactionnel a été porté à reflux pendant 5 heures, puis laissé refroidir à température ambiante pour être filtré et concentré sous vide. On a ainsi obtenu 1,57 g (1,57 g, huile jaune légèrement verte) correspondant au composé 2.

Les spectres RMN $^1H$, $^{13}C$ et de masse sont conformes à la structure du produit attendu.

**Exemple 2 : Synthèse du 1-(α,β)-D-xylopyranosyl, 4-(4-hydroxy,3-méthoxy-phenyl) butane-2-ol**

**(Composé 1)**

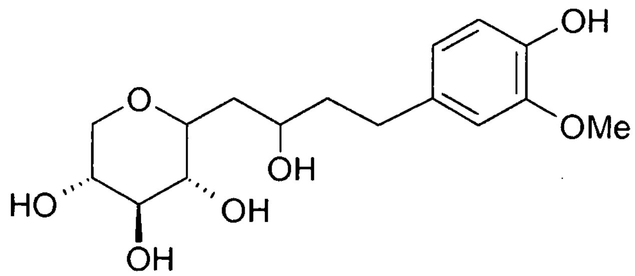

Le composé 2 obtenu à l'exemple 1 (1.57 g, 1 éq) a été solubilisé dans l'éthanol (20 ml) puis le borohydrure de sodium (0.182 g, 1 éq) a été additionné par petites portions. Le milieu a été agité à température ambiante pendant 12 heures. Puis de l'acétone a été additionné pour détruire le borohydrure de sodium en excès, puis de l'acide chlorhydrique aqueux (1 N) pour ajuster le pH à 2. Le milieu réactionnel a été concentré sous vide, et repris dans l'eau, puis lavé deux fois avec de l'acétate d'éthyle. La phase aqueuse a ensuite été extraite avec du butanol, et les phases organiques ont été réunies et concentrées sous vide. Le produit brut obtenu a été purifié sur gel de silice pour obtenir 0,53g (rendement de 34%) d'une huile légèrement jaune correspondant au composé 1 pur.

Les spectres RMN $^1H$, $^{13}C$ et de masse sont conformes à la structure du produit attendu.

**<u>Exemple 3 : Synthèse du (3E)-1-(α,β)-D-xylopyranosyl, 4-phényl but-3-ène-2-one</u>**

**(Composé 5)**

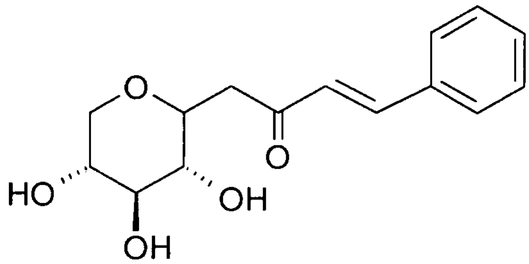

Dans 50 ml d'éthanol, on a fait réagir 2 g (10,5 mmol) de C-β-D-xylopyranoside-n-propane-2-one, avec 1,2 g (11,6 mmole) de benzaldéhyde, puis on a ajouté 5,8 ml (15,8 mmol) d'une solution de méthylate de sodium à 21 % dans l'éthanol. Le milieu a été agité pendant une heure puis la réaction a été stoppée par addition d'une solution saturée en chlorure d'ammonium.

Le milieu réactionnel a ensuite été concentré sous vide et repris dans l'eau. La phase aqueuse a extraite avec du butanol. Les phases organiques ont été séchées et concentrées sous vide. Le produit brut obtenu a été purifié sur gel de silice pour obtenir 700 mg (rendement 25%) du produit pur attendu sous forme d'un solide blanc.

Les spectres RMN $^1H$, $^{13}C$ et de masse sont conformes à la structure du produit attendu.

**<u>Exemple 4 : Synthèse du 1-(α,β)-D-xylopyranosyl, 4-phényl butane-2-one</u>**

**(composé 4):**

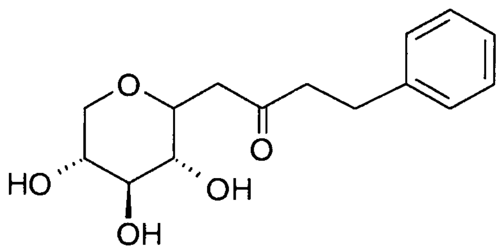

Dans un ballon muni d'un agitateur magnétique, on a introduit 12 g du composé 5 de l'exemple 3 dans 70 ml de méthanol et on a ajouté 0,5 g de palladium sur charbon à 10 % molaire. Le mélange réactionnel a été agité et placé sous pression d'hydrogène (100000 Pa soit 1 bar) pendant 15 heures. Ensuite, l'insoluble a été filtré sur une couche de célite, et le filtrat a été évaporé sous vide. On a obtenu 12 g (rendement 99%) de composé 4 sous forme d'un produit solide blanc cassé.

**Exemple 5 : Synthèse du : (composé 4) : 1-(α,β)-D-xylopyranosyl, 4-phényl butane-2-ol**

**Composé 9**

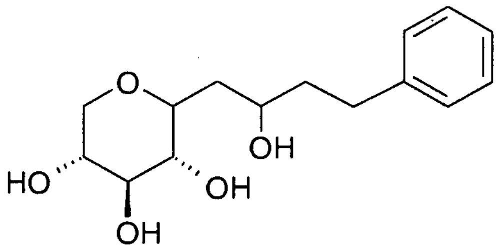

Dans un ballon de 250 ml, muni d'un agitateur magnétique, on a introduit, sous azote, 12 g (0,043 mol) du composé 4 de l'exemple 4 dans 100 ml de méthanol. 1,954 g (0,0514 mole, 1,2 éq.) de borohydrure de sodium a ensuite été ajouté en petites portions sous forte agitation à -20°C. A la fin de l'addition, l'agitation a été maintenue pendant 10 minutes à 0°C, puis le mélange a été hydrolysé à cette température, à l'aide d'HCl (3N) jusqu'à pH = 4. Le mélange réactionnel a ensuite été évaporé à sec sous vide. Le résidu obtenu a été dilué par l'acétone, puis filtré pour éliminer les sels formés et le filtrat a été évaporé puis séché sous vide. Le produit brut obtenu a été purifié par flash chromatographie sur une colonne de silice (éluant : éther, puis acétone) pour récupérer le composé 9 attendu (rendement 72 %) sous forme de cristaux blancs cassés.

**Exemple 6 : Synthèse du 1-(α,β)-D-xylopyranosyl, 4-(2-hydroxyphényl) butane-2-ol**

**composé 8**

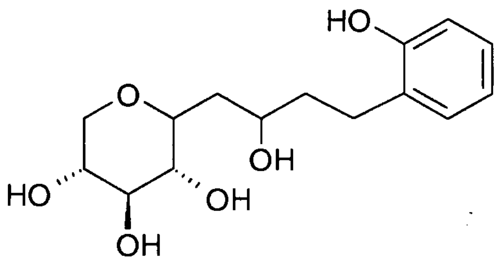

a) Synthèse du composé A de formule :

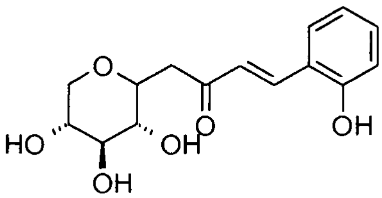

Composé A

La C-β-D-xylopyranoside-n-propane-2-one (1équiv., 4.00g) en solution dans un mélange éthanol (4.5ml) + eau (3.5ml) a été traitée par une solution aqueuse de soude (3.5équiv., 2.94 g, dans 27 ml d'eau) à température ambiante. Après 2 heures d'agitation, le salicylaldéhyde (1.2équiv., 2.7 ml) a été additionné goutte-à-goutte à température ambiante. Après 20 heures d'agitation, le pH a été ajusté à 6 puis le mélange réactionnel a été concentré. L'analyse par CCM du brut réactionnel a révélé la formation d'un nouveau produit largement majoritaire (Rf = 0.38 pour $CH_2Cl_2$/MeOH : 80/20)

La purification de ce résidu par chromatographie sur colonne de silice (dépôt solide, $CH_2Cl_2$/MeOH : 80/20) permet de récupérer le composé A attendu sous la forme d'une poudre orange (4.27g, rdt = 69%).

b) Synthèse du composé B de formule :

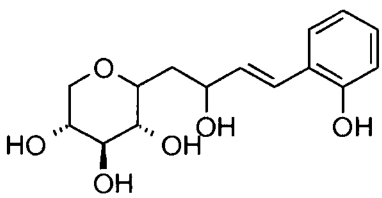

Composé B

Le composé A (1 équiv., 4.2g) a été mis en solution dans méthanol (150 ml) puis a été traité par $NaBH_4$ (2équiv., 1.1g), ajouté à température ambiante, par portions pour limiter l'effervescence qui se produit. Le mélange réactionnel a été agité pendant 3 heures, durée au bout de laquelle une analyse par CCM du mélange réactionnel a révélé la disparition complète du produit de départ. L'excès de réducteur a été neutralisé par addition d'une solution aqueuse saturée de chlorure d'ammonium (20mL) puis le mélange réactionnel a été concentré.

Le brut réactionnel a été purifié par chromatographie sur colonne de silice (Rf = 0.45 pour $CH_2Cl_2$/MeOH : 80/20) pour obtenir le composé B..

c) Synthèse du 1-(α,β)-D-xylopyranosyl, 4-(2-hydroxyphényl) butane-2-ol

Le composé B (120mg) a été mis en solution dans 10 ml d'éthanol, auxquels 3 gouttes d'eau ont été ajoutés, puis a été hydrogéné à 30°C en utilisant du catalyseur Pd/C 10%. Après évaporation, le produit attendu est obtenu sous la forme d'une huile beige (60mg, rdt = 49%).

**<u>Exemple 7</u>**

Etude de l'effet de dérivés C-glycosides sur la synthèse des glycosaminoglycannes sulfatés sur fibroblastes et sur kératinocytes :

L'étude est faite par mesure de l'incorporation de glucosamine radioactive dans la matrice néosynthétisée par des cultures de fibroblastes dermiques humains normaux ou par des kératinocytes. L'incorporation de glucosamine radioactive indique une néosynthèse spécifique de glycosaminoglycannes via une incorporation de la forme acétylée de cette glucosamine.

Les cultures de fibroblastes sont effectuées selon les méthodes classiques de culture cellulaire, à savoir en milieu DMEM vendu par la société Gibco, en présence de L-glutamine (2 mM), de pénicilline (50 UI/ml) et de 10% de sérum de veau foetal (Gibco).

Les cultures de kératinocytes sont effectuées en milieu Keratinocyte-SFM vendu par la société Gibco , en présence d'EGF (Epidermal Growth Factor) (0,25 ng/ml), d'extrait pituitaire (25 μg/ml) et de gentamycine (25 μg/ml).

Les fibroblastes et les kératinocytes ont été cultivés en plaques de 96 puits. A confluence, le milieu de culture a été remplacé par du milieu de culture adéquat contenant ou non (témoin) le composé à tester ou la référence, puis les cellules ont été incubées pendant 48 heures. Le marqueur radioactif 35 S-sulfate a été ajouté (40 μCi final) et les cellules ont été incubées pendant 24 heures supplémentaires. Toutes les conditions ont été réalisées 3 fois.

Les glycosaminoglycanes de la matrice extracellulaire ont été extraits avec un volume tampon chaotropique (Tris/HCl 50 mM, guanidine 4 M, EDTA 5 mM, pH 8,0).

Les GAGs sulfatés ont été purifiés par chromatographie d'échanges d'ions : absorption des molécules anioniques sur billes de Q-Sépahrose dans des conditions de forte stringence, désorption des molécules peu et moyennement anioniques avec uns solution urée 6 M NaCl 0,2 mM, puis lavages.

La radioactivité incorporée dans les molécules très cationiques restées sur le support (GAGs majoritairement)

a été mesurée par scintillation liquide.

Les résultats sont évalués par apport à un témoin constitué par des cellules n'ayant pas été traitées avec un composé de formule (I).

Un témoin positif (TGFβ à 10 ng/ml) connu pour stimuler la synthèse des GAGs est introduit dans le test effectéu sur fibroblastes comme référence positive.

Un témoin positif ($CaCl_2$ à 1,4 mM) connu pour stimuler la synthèse des GAGs est introduit dans le test effectué sur kératinocytes comme référence positive .

Les résultats sont exprimés en pourcentage de variation de la synthèse des glycosaminoglycanes par rapport au témoin.

On a effectué 3 essais pour le composé testé.

Les comparaisons des résultats obtenus pour un composé testé ont été réalisés à l'aide du test de Student.

L'écart type de la moyenne (esm) a été calculé selon la relation suivante :

$$esm = \text{écart type} / (n)^{1/2}$$

n étant le nombre d'essais effectués.

Les résultats sont présentés dans le tableau suivant :

| **Traitement sur fibroblastes** | | Valeur moyenne | esm | n | % | p |
|---|---|---|---|---|---|---|
| Contrôle (milieu de culture) | | 2332 | 7 | 3-3 | 100-100 | 0,01 à 0,05 |
| Contrôle positif (TGFβ) | | 4062 | 9 | 3-3 | 174 | <0,001 |
| Composé de l'exemple 2 | 40 μM<br>120 μM | 8130<br>15318 | 28<br>22 | 3<br>3 | 351<br>657 | <0,001<br><0,001 |
| **Traitement sur kératinocytes** | | Valeur moyenne | esm | n | % | p |
| Contrôle (milieu de culture) | | 4526 | 4 | 3-3 | 100-100 | 0,01 à 0,05 |
| Contrôle positif ($CaCl_2$) | | 5273 | 3 | 3-3 | 117 | 0,001 à 0,01 |
| Composé de l'exemple 2 | 0,37 mM<br>1,1 mM | 31999<br>36795 | 23<br>34 | 3<br>3 | 707<br>813 | <0,001<br><0,001 |
| Les valeurs mesurées sont données en coups par minute (cpm)<br>n : nombre d'essais effectués<br>p : intervalle de confiance<br>esm : écart type de la moyenne | | | | | | |

Les résultats obtenus montrent de façon significative que le composé de l'exemple 2 (Composé 1) est très efficace pour augmenter la synthèse des GAGs sulfatés.

Cette activité permet d'attribuer à ce composé une activité biologique sur la fermeté de la peau et ainsi d'estomper les signes du vieillissement cutané.

Dans un autre plan d'expérience, on a évalué les composés des exemples 5 (composé 9) et 6 (composé 8).

| **Traitement sur fibroblastes** | | Valeur moyenne | esm | n | % |
|---|---|---|---|---|---|
| Contrôle (milieu de culture) | | 2518 | 2 | 2 | 100 |
| Composé 8 de l'exemple 6 | 0,1 mM<br>1 mM | 2938<br>10371 | 13<br>34 | 2<br>2 | 117<br>412 |
| Composé 9 de l'exemple 5 | 0,1 mM<br>1 mM | 10838<br>23620 | 9<br>21 | 2<br>2 | 430<br>938 |
| **Traitement sur kératinocytes** | | Valeur moyenne | esm | n | % |
| Contrôle (milieu de culture) | | 4526 | 4 | 2 | 100 |
| Composé 8 de l'exemple 6 | 0,1 mM<br>1 mM | 40552<br>85456 | 40<br>47 | 2<br>2 | 316<br>665 |

(suite)

| Traitement sur kératinocytes | | Valeur moyenne | esm | n | % |
|---|---|---|---|---|---|
| Composé 9 de l'exemple 5 | 0,1 μM | 24106 | 5 | 2 | 563 |
| | 1 μM | 99041 | 112 | 2 | 547 |

Les résultats obtenus montrent de façon significative que les composés 1, 8 et 91 sont très efficaces pour augmenter la synthèse des GAGs sulfatés.
Cette activité permet d'attribuer à ce composé une activité biologique sur la fermeté de la peau et ainsi d'estomper les signes du vieillissement cutané.

**Exemple 7**

On prépare une crème de soin du visage de type émulsion huile-dans-eau, comprenant (% en poids) :

| | |
|---|---|
| composé de l'exemple 2 | 0,005% |
| stéarate de glycérol | 2% |
| polysorbate 60 (Tween 60 de ICI) | 1% |
| acide stéarique | 1,4% |
| triéthanolamine | 0,7% |
| carbomer | 0,4% |
| fraction liquide du beurre de karité | 12% |
| perhydrosqualène | 12% |
| antioxydant | qs |
| parfum, conservateur | qs |
| eau | qsp 100% |

Une composition similaire est préparée avec le composé des exemples 1 ou 3 à 5.
La composition appliquée sur le visage permet de renforcer la fermeté de la peau et ainsi d'estomper les signes du vieillissement cutané.

**Exemple 8**

On prépare un gel anti-âge pour la peau comprenant (% en poids) :

| | |
|---|---|
| composé de l'exemple 2 | 2% |
| hydroxypropylcellulose (Klucel H de Hercules) | 1% |
| antioxydant | qs |
| parfum, conservateur | qs |
| isopropanol | 40% |
| eau | qsp 100% |

Une composition similaire est préparée avec le composé des exemples 1 ou 3 à 5.
La composition appliquée sur le visage permet de renforcer la fermeté de la peau et ainsi d'estomper les signes du vieillissement cutané.

**Revendications**

1. Composés de formule (I):

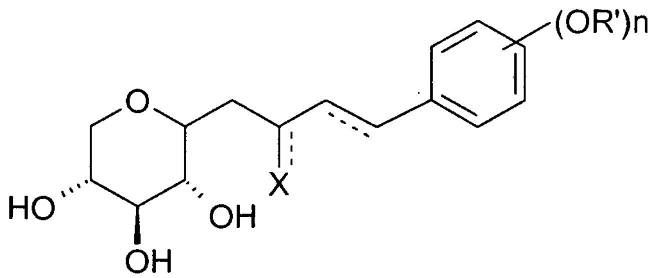

dans laquelle :

X désigne un groupement -OH ou =O ;
n est un entier allant de 0 à 5 ;
R' désigne :

un atome d'hydrogène ou
un radical alkyle linéaire en $C_1$-$C_4$ ou ramifié en $C_3$-$C_4$, ou un radical hydrocarboné insaturé en $C_3$-$C_4$ ,
lorsque n ≥ 2 , deux groupements adjacents -OR' peuvent former ensemble radical divalent -O-$CH_2$-O- ;
sous réserve que lorsque X = OH, le composé ne comporte pas de double liaison éthylénique en position alpha du carbone portant cet OH ;
ainsi que leurs sels, et leurs isomères optiques.

**2.** Composés selon la revendication 1, dans lesquels:

X désigne un groupement -OH ou =O ;
n est un entier allant de 0 à 3 ;
R' désigne :

un atome d'hydrogène ou
un radical alkyle linéaire en $C_1$-$C_4$ , notamment un radical méthyle ou n-butyle ;
ou un radical -CO-$CH_3$ ;
lorsque n ≥ 2 , deux groupements adjacents -OR' peuvent former ensemble radical divalent -O-$CH_2$-O-.

**3.** Composés selon l'une des revendications précédentes, dans lesquels :

X désigne un groupement -OH ou =O ;
n est un entier allant de 0 à 3 ;
R' désigne :

un atome d'hydrogène ou
un radical un radical méthyle.

**4.** Composés selon l'une des revendications précédentes, choisi parmi les composés suivants :

- 1-(α,β)-D-xylopyranosyl, 4-(4-hydroxy,3-méthoxy-phenyl) butane-2-ol ;
- 1-(α,β)-D-xylopyranosyl, 4-(4-hydroxy,3-méthoxy-phényl) butane-2-one ;
- 1-(α,β)-D-xylopyranosyl, 4-(4-hydroxyphényl) butane-2-ol ;
- 1-(α,β)-D-xylopyranosyl, 4-phényl butane-2-one ;
- (3E)-1-(α,β)-D-xylopyranosyl, 4-phényl but-3-ène-2-one ;
- (3E)-1-(α,β)-D-xylopyranosyl, 4-[4-hydroxy, (3,5)-diméthoxy-phényl] but-3-ène-2-one ;
- 1-(α,β)-D-xylopyranosyl, 4-[(3,4)-méthylènedioxyphenyl] butane-2-ol ;
- 1-(α,β)-D-xylopyranosyl, 4-(2-hydroxyphényl) butane-2-ol
- 1-(α,β)-D-xylopyranosyl, 4-phényl butane-2-ol

et leurs sels ou leurs solvates.

5. Composition cosmétique ou pharmaceutique comprenant, dans un milieu physiologiquement acceptable, au moins un composé de formule (I) tel que défini à l'une quelconque des revendications 1 à 4.

6. Composition selon la revendication précédente, dans laquelle le composé de formule (I) est présent, seul ou en mélange, en une quantité comprise entre 0,01 et 10% en poids, de préférence entre 0,1 à 5% en poids, notamment entre 0,5 à 3% en poids, par rapport au poids total de la composition.

7. Composition selon l'une des revendications 5 ou 6, dans laquelle le milieu physiologiquement acceptable comprend au moins un adjuvant cosmétique choisi parmi l'eau; les solvants organiques, notamment les alcools en $C_1$-$C_6$ et les esters d'acide carboxylique en $C_2$-$C_{10}$ ; les huiles hydrocarbonées, les huiles siliconées, les huiles fluorées, les cires, les pigments, les charges, les colorants, les tensioactifs, les émulsionnants, les actifs cosmétiques ou dermatologiques, les filtres UV, les polymères filmogènes, les gélifiants hydrophiles ou lipophiles, les épaississants, les conservateurs, les parfums, les bactéricides, les absorbeurs d'odeur, les antioxydants.

8. Composition selon l'une des revendications 5 à 7, dans laquelle le milieu physiologiquement acceptable comprend au moins un composé choisi parmi: les agents desquamants; les agents hydratants; les agents dépigmentants ou propigmentants; les agents anti-glycation; les inhibiteurs de NO-synthase; les agents stimulant la synthèse de macromolécules dermiques ou épidermiques et/ou empêchant leur dégradation; les agents stimulant la prolifération des fibroblastes et/ou des kératinocytes ou stimulant la différenciation des kératinocytes; les agents myorelaxants et/ou les agents dermo-décontractants; les agents tenseurs; les agents anti-pollution et/ou anti-radicalaire; les agents agissant sur la microcirculation; les agents agissant sur le métabolisme énergétique des cellules; et leurs mélanges.

9. Composition selon l'une des revendications 6 à 8, se présentant sous forme d'une composition anti-âge, notamment de soin destinée à lutter contre les signes extérieurs du vieillissement cutané.

10. Procédé de traitement cosmétique non thérapeutique de la peau, comprenant l'application sur la peau, d'une composition cosmétique telle que définie à l'une des revendications 6 à 9.

11. Procédé selon la revendication précédente dans lequel la composition est appliquée sur la peau mature et/ou ridée.

12. Utilisation non thérapeutique d'une composition cosmétique telle que définie à l'une des revendications 6 à 9, ou d'un composé de formule (I) tel que défini à l'une des revendications 1 à 4, pour améliorer la fermeté de la peau et/ou pour améliorer la structure de la jonction dermo-épidermique et/ou pour renforcer la cohésion entre le derme et l'épiderme.

13. Utilisation non thérapeutique d'une composition cosmétique telle que définie définie à l'une des revendications 6 à 9 ou d'un composé de formule (I) tel que défini à l'une des revendications 1 à 4, pour prévenir et/ou traiter les signes cutanés du vieillissement, notamment les signes cutanés choisis parmi la peau ridée, la peau présentant une altération de ses propriétés viscoélastiques ou biomécaniques, la peau présentant une altération dans la cohésion de ses tissus, la peau amincie, et la peau présentant une altération de son aspect de surface.

**Patentansprüche**

1. Verbindungen der Formel (I):

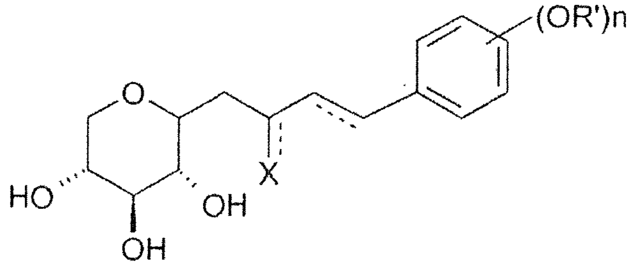

worin:

X für eine -OH- oder =O-Gruppe steht;
n für eine ganze Zahl im Bereich von 0 bis 5 steht;
R' für:

ein Wasserstoffatom oder
einen linearen $C_1$-$C_4$- oder verzweigten $C_3$-$C_4$-Alkylrest oder einen ungesättigten $C_3$-$C_4$-Kohlenwasserstoffrest
steht,
im Fall von n ≥ 2 zwei benachbarte -OR'-Gruppen zusammen einen zweiwertigen -O-$CH_2$-O- Rest bilden können;
mit der Maßgabe, dass im Fall von X = OH die Verbindung keine ethylenische Doppelbindung in alpha-Position zu dem dieses OH tragenden Kohlenstoff enthält;
sowie Salze davon und optische Isomere davon.

**2.** Verbindungen nach Anspruch 1 worin:

X für eine -OH- oder =O-Gruppe steht;
n für eine ganze Zahl im Bereich von 0 bis 3 steht;
R' für:

ein Wasserstoffatom oder
einen linearen $C_1$-$C_4$-Alkylrest, insbesondere einen Methyl- oder n-Butylrest
oder einen -CO-$CH_3$-Rest
steht,
im Fall von n ≥ 2 zwei benachbarte -OR'-Gruppen zusammen einen zweiwertigen -O-$CH_2$-O- Rest bilden können.

**3.** Verbindungen nach einem der vorhergehenden Ansprüche, worin:

X für eine -OH- oder =O-Gruppe steht;
n für eine ganze Zahl im Bereich von 0 bis 3 steht;
R' für:

ein Wasserstoffatom oder
einen Methylrest
steht.

**4.** Verbindungen nach einem der vorhergehenden Ansprüche, ausgewählt aus den folgenden Verbindungen:

- 1-(α,β)-D-Xylopyranosyl-4-(4-hydroxy-3-methoxyphenyl)butan-2-ol;
- 1-(α,β)-D-Xylopyranosyl-4-(4-hydroxy-3-methoxyphenyl)butan-2-on;
- 1-(α,β)-D-Xylopyranosyl-4-(4-hydroxyphenyl)-butan-2-ol;
- 1-(α,β)-D-Xylopyranosyl-4-phenylbutan-2-on;
- (3E)-1-(α,β)-D-Xylopyranosyl-4-phenylbut-3-en-2-on;
- (3E)-1-(α,β)-D-Xylopyranosyl-4-[4-hydroxy-(3,5)-dimethoxyphenyl]but-3-en-2-on;
- 1-(α,β)-D-Xylopyranosyl-4-[(3,4)-methylendioxyphenyl]butan-2-ol;
- 1-(α,β)-D-Xylopyranosyl-4-(2-hydroxyphenyl)-butan-2-ol;
- 1-(a,β)-D-Xylopyranosyl-4-phenylbutan-2-ol;

und Salzen davon oder Solvaten davon.

**5.** Kosmetische oder pharmazeutische Zusammensetzung, die in einem physiologisch unbedenklichen Medium mindestens eine Verbindung der Formel (I) gemäß einem der Ansprüche 1 bis 4 umfasst.

**6.** Zusammensetzung nach dem vorhergehenden Anspruch, in der die Verbindung der Formel (I) allein oder als Gemisch in einer Menge zwischen 0,01 und 10 Gew.-%, vorzugsweise zwischen 0,1 und 5 Gew.-%, insbesondere zwischen 0,5 und 3 Gew.-%, bezogen auf das Gesamtgewicht der Zusammensetzung, vorliegt.

7. Zusammensetzung nach Anspruch 5 oder 6, in der das physiologisch unbedenkliche Medium mindestens einen kosmetischen Hilfsstoff, der aus Wasser; organischen Lösungsmitteln, insbesondere $C_1$-$C_6$-Alkoholen und $C_2$-$C_{10}$-Carbonsäureestern; Kohlenwasserstoffölen, Silikonölen, fluorierten Ölen, Wachsen, Pigmenten, Füllstoffen, Farbmitteln, Tensiden, Emulgatoren, kosmetischen oder dermatologischen Wirkstoffen, UV-Schutzmitteln, filmbildenden Polymeren, hydrophilen oder lipophilen Geliermitteln, Verdickungsmitteln, Konservierungsmitteln, Duftstoffen, Bakteriziden, Geruchsabsorptionsmitteln und Antioxidantien ausgewählt ist, umfasst.

8. Zusammensetzung nach einem der Ansprüche 5 bis 7, wobei das physiologisch unbedenkliche Medium mindestens eine Verbindung, die aus Entschuppungsmitteln; Feuchtigkeitsmitteln; depigmentierenden oder propigmentierenden Mitteln; Antiglykierungsmitteln; NO-Synthase-Inhibitoren; Mitteln zur Stimulierung der Synthese von dermalen oder epidermalen Makromolekülen und/oder zur Verhinderung ihrer Degradation; Mitteln zur Stimulierung der Fibroblasten- und/oder Keratinozytenproliferation oder zur Stimulierung der Keratinozytendifferenzierung; Muskelrelaxantien und/oder Hautdekontraktionsmitteln; Straffungsmitteln; Mitteln gegen Schadstoffe und/oder Radikale; Mitteln, die auf die Mikrozirkulation wirken; Mitteln, die auf den Energiestoffwechsel von Zellen wirken; und Mischungen davon ausgewählt ist, umfasst.

9. Zusammensetzung nach einem der Ansprüche 6 bis 8, die in Form einer Alterungsschutzzusammensetzung, insbesondere einer Pflegezusammensetzung zur Bekämpfung der äußeren Zeichen der Hautalterung, vorliegt.

10. Verfahren zur nichttherapeutischen kosmetischen Behandlung der Haut, bei dem man auf die Haut eine kosmetische Zusammensetzung gemäß einem der Ansprüche 6 bis 9 aufbringt.

11. Verfahren nach dem vorhergehenden Anspruch, bei dem man die Zusammensetzung auf reife und/oder faltige Haut aufbringt.

12. Nichttherapeutische Verwendung einer kosmetischen Zusammensetzung gemäß einem der Ansprüche 6 bis 9 oder einer Verbindung der Formel (I) gemäß einem der Ansprüche 1 bis 4 zur Verbesserung der Straffheit der Haut und/oder zur Verbesserung der Struktur der Dermis-Epidermis-Verbindung und/oder zur Verstärkung der Kohäsion zwischen der Dermis und der Epidermis.

13. Nichttherapeutische Verwendung einer kosmetischen Zusammensetzung gemäß einem der Ansprüche 6 bis 9 oder einer Verbindung der Formel (I) gemäß einem der Ansprüche 1 bis 4 zur Prävention und/oder Behandlung der Hautalterungszeichen, insbesondere der aus faltiger Haut, Haut mit einer Beeinträchtigung ihrer viskoelastischen oder biomechanischen Eigenschaften, Haut mit einer Beeinträchtigung der Kohäsion ihrer Gewebe, dünner gewordener Haut und Haut mit einer Beeinträchtigung ihres Oberflächenaussehens ausgewählten Hautzeichen.

**Claims**

1. Compounds of formula (I):

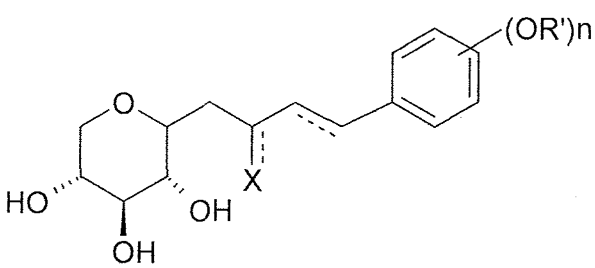

in which:

X denotes an -OH or =O group;
n is an integer ranging from 0 to 5;
R' denotes:

a hydrogen atom or

a linear $C_1$-$C_4$ or branched $C_3$-$C_4$ alkyl radical, or an unsaturated $C_3$-$C_4$ hydrocarbon-based radical,
when $n \geq 2$, two adjacent -OR' groups can together form the divalent radical -O-$CH_2$-O-;
with the proviso that, when X = OH, the compound does not comprise an ethylenic double bond in the alpha-position of the carbon bearing this OH;
and also the salts thereof, and the optical isomers thereof.

**2.** Compounds according to Claim 1, in which:

X denotes an -OH or =O group;
n is an integer ranging from 0 to 3;
R' denotes:

a hydrogen atom or
a linear $C_1$-$C_4$ alkyl radical, in particular a methyl or n-butyl radical;
or a -CO-$CH_3$ radical;
when $n \geq 2$, two adjacent -OR' groups can together form the divalent radical -O-$CH_2$-O-.

**3.** Compounds according to either of the preceding claims, in which:

X denotes an -OH or =O group;
n is an integer ranging from 0 to 3;
R' denotes:

a hydrogen atom or
a methyl radical.

**4.** Compounds according to one of the preceding claims, chosen from the following compounds:

- 1-($\alpha$,$\beta$)-D-xylopyranosyl-4-(4-hydroxy-3-methoxyphenyl)butan-2-ol;
- 1-($\alpha$,$\beta$)-D-xylopyranosyl-4-(4-hydroxy-3-methoxyphenyl)butan-2-one;
- 1-($\alpha$,$\beta$)-D-xylopyranosyl-4-(4-hydroxyphenyl)butan-2-ol;
- 1-($\alpha$,$\beta$)-D-xylopyranosyl-4-phenylbutan-2-one;
- (3E)-1-($\alpha$,$\beta$)-D-xylopyranosyl-4-phenylbut-3-en-2-one;
- (3E)-1-($\alpha$,$\beta$)-D-xylopyranosyl-4-[4-hydroxy-(3,5)-dimethoxyphenyl]but-3-en-2-one;
- 1-($\alpha$,$\beta$)-D-xylopyranosyl-4-[(3,4)-methylenedioxyphenyl]butan-2-ol;
- 1-($\alpha$,$\beta$)-D-xylopyranosyl-4-(2-hydroxyphenyl)butan-2-ol;
- 1-($\alpha$,$\beta$)-D-xylopyranosyl-4-phenylbutan-2-ol;

and the salts thereof or the solvates thereof.

**5.** Cosmetic or pharmaceutical composition comprising, in a physiologically acceptable medium, at least one compound of formula (I) as defined in any one of Claims 1 to 4.

**6.** Composition according to the preceding claim, in which the compound of formula (I) is present, alone or as a mixture, in an amount of between 0.01% and 10% by weight, preferably between 0.1% and 5% by weight, in particular between 0.5% and 3% by weight, relative to the total weight of the composition.

**7.** Composition according to either of Claims 5 and 6, in which the physiologically acceptable medium comprises at least one cosmetic adjuvant chosen from water; organic solvents, in particular $C_1$-$C_6$ alcohols and $C_2$-$C_{10}$ carboxylic acid esters; hydrocarbon-based oils, silicone oils, fluoro oils, waxes, pigments, fillers, dyes, surfactants, emulsifiers, cosmetic or dermatological active agents, UV-screening agents, film-forming polymers, hydrophilic or lipophilic gelling agents, thickeners, preservatives, fragrances, bactericides, odor absorbers and antioxidants.

**8.** Composition according to one of Claims 5 to 7, in which the physiologically acceptable medium comprises at least one compound chosen from: desquamating agents; moisturizing agents; depigmenting or propigmenting agents; anti-glycation agents; NO-synthase inhibitors; agents which stimulate the synthesis of dermal or epidermal macromolecules and/or prevent degradation thereof; agents which stimulate fibroblast and/or keratinocyte proliferation or

stimulate keratinocyte differentiation; muscle relaxants and/or dermodecontracting agents; tensioning agents; anti-pollution agents and/or free-radical scavengers; agents which act on the microcirculation; agents which act on the energy metabolism of cells; and mixtures thereof.

**9.** Composition according to one of Claims 6 to 8, which is in the form of an anti-aging, in particular care, composition intended for combating the external signs of aging of the skin.

**10.** Method for nontherapeutic cosmetic treatment of the skin, comprising the application, to the skin, of a cosmetic composition as defined in one of Claims 6 to 9.

**11.** Method according to the preceding claim, in which the composition is applied to mature and/or wrinkled skin.

**12.** Nontherapeutic use of a cosmetic composition as defined in one of Claims 6 to 9, or of a compound of formula (I) as defined in one of Claims 1 to 4, for improving the firmness of the skin and/or for improving the structure of the dermal-epidermal junction and/or for reinforcing the cohesion between the dermis and the epidermis.

**13.** Nontherapeutic use of a cosmetic composition as defined in one of Claims 6 to 9, or of a compound of formula (I) as defined in one of Claims 1 to 4, for preventing and/or treating the signs of aging on the skin, in particular the signs on the skin chosen from wrinkled skin, skin exhibiting a detrimental modification of its viscoelastic or biomechanical properties, skin exhibiting a detrimental modification in the cohesion of its tissues, thinned skin, and skin exhibiting a detrimental modification of its surface appearance.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 02051828 A
- WO 2006128738 A